# EUROPÄISCHE PATENTSCHRIFT

(11) **EP 3 487 869 B1**
(45) Veröffentlichungstag und Bekanntmachung des Hinweises auf die Patenterteilung: **06.09.2023**
(21) Anmeldenummer: 16754409.7
(22) Anmeldetag: 25.07.2016
(51) Int. Cl.: C07K 1/32, C12N 15/10, C12Q 1/68

(54) **VERFAHREN ZUR ANREICHERUNG VON BIOMOLEKÜLEN UND ZUR ENTFERNUNG DER BIOMOLEKÜLE AUS EINER BIOLOGISCHEN PROBE**
METHOD FOR ENRICHING BIOMOLECULES AND FOR REMOVING THE BIOMOLECULES FROM A BIOLOGICAL SAMPLE
PROCÉDÉ D'ENRICHISSEMENT EN BIOMOLÉCULES ET DE SUPPRESSION DE BIOMOLÉCULES D'UN ÉCHANTILLON BIOLOGIQUE

(43) Veröffentlichungstag der Anmeldung: 29.05.2019
(73) Patentinhaber: IST Innuscreen GmbH, 13125 Berlin (DE)
(72) Erfinder: HILLEBRABD, Timo, 15366 Hoppegarten (DE)
(74) Vertreter: Wehlan, Helmut
(86) Internationale Anmeldenummer: PCT/EP2016/067667
(87) Internationale Veröffentlichungsnummer: WO 2018/019360

(56) Entgegenhaltungen:
- EP-A1- 2 261 231
- WO-A1-2009/135936
- WO-A1-2013/124863
- DE-A1-102015 215 894

## Beschreibung

Gegenstand der Erfindung ist ein einfaches Verfahren zur Anreicherung von Biomolekülen und zur Entfernung (Isolierung) der Biomoleküle aus einer Probe für eine nachfolgende Untersuchung dieser Biomoleküle bzw. für eine weitere Bearbeitung. Zu den Biomolekülen, die angereichert werden sollen, zählen zellfreie Nukleinsäuren, Viren oder subzelluläre Mikropartikel (z.B. Exosomen). Das Verfahren ist ein verbessertes und einfacheres Verfahren als das in der Patentschrift DE 10 2008 023 297 B4 beschriebene Verfahren. Das Dokument WO 2013/124863 A1 offenbart ein Verfahren zur Anreicherung/ Abtrennung von Biomolekülen mit Hilfe von mit Alginat beschichtete Magnetpartikeln.

Es ist bekannt, dass sich in zellfreien Körperflüssigkeiten sog. frei zirkulierende Nukleinsäuren, Exosomen sowie im Falle einer viralen Infektion auch Viruspartikel befinden. Alle diese unterschiedlichen Biomoleküle sind von großer Bedeutung für die Diagnostik von Erkrankungen. Insbesondere zellfreie Nukleinsäuren sowie Exosomen spielen dabei eine immer bedeutendere Rolle. Allen diesen Biomolekülen ist auch gemeinsam, dass sie nur in sehr geringen Konzentrationen in Körperflüssigkeiten vorliegen. Dies erschwert ihre diagnostische Nutzung. Der einzige Ausweg besteht darin, größere Probenvolumina zu bearbeiten, um final eine ausreichende Menge an Biomolekülen verfügbar zu haben. Als wichtige Ausgangsproben kommen z.B. Körperflüssigkeiten wie Blutplasma oder Serum oder auch Urin in Frage.

Es gibt zurzeit nur wenige Verfahren, die es erlauben, diese Biomoleküle aus einer großvolumigen Probe anzureichern bzw. zu isolieren bzw. nachfolgend die angereicherten Biomoleküle für die Extraktion von Nukleinsäuren zu verwenden.

So gibt es zum Beispiel für die Anreicherung von Exosomen aus einer biologischen Probe die Anwendung von Ultrazentrifugationstechniken. Dabei kommt es zur Ansammlung von Exosomen am Boden des Reaktionsgefäßes. Diese Methode ist an eine Ultrazentrifuge gebunden und darüber hinaus zeitaufwendig und für die Routinediagnostik ungeeignet. Weiterhin wird auch die Technik der Ultrafiltration eingesetzt. Auch diese Methode ist zeitaufwendig und sehr teuer. Alternative Verfahren bestehen in einer Immunopräzipitation der Exosomen mittels einer Immunoplate oder mittels Immunobeads. Auch diese Art der Anreicherung der Exosomen ist zeitaufwendig und auf Grund der einzusetzenden Reagenzien störanfällig sowie teuer. Darüber hinaus können bei dieser Technologie lediglich 200 - 500 µl an Probe eingesetzt werden. Für die Anreicherung von Viren können ebenfalls Ultrazentrifugationstechniken eingesetzt werden. Alternative Verfahren bestehen in der Präzipitation von Viruspartikeln mittels Polyethylenglycol/ Natriumchlorid und einer nachfolgenden Zentrifugation (Yamamoto et al., Virology 40(1970) 734; Morandi et al., J.Clin.Microbiol. 36 (1998) 1543-1538). Dabei nutzt man verschiedene Mischungen von PEG und Natriumchlorid und mischt diese Reagenzien mit der biologischen Probe. Nachfolgend wird der Ansatz bei Kälte längere Zeit inkubiert und anschließend die Virus(Protein)- NaCl/ PEG- Präzipitate durch Zentrifugation gewonnen. Auch diese Verfahren sind aufwendig und benötigen viel Zeit. Problematisch ist darüber hinaus die Weiterbearbeitung der Präzipitate für die Isolierung der viralen Nukleinsäuren. Oftmals lassen sich die Präzipitate nur sehr schwer wieder in Lösung bringen. Dies beeinflusst die Effizienz und die Qualität der Nukleinsäureisolierung erheblich. Die Patentschrift DE 19856415 C2 beschreibt ein Verfahren, welches sich der bekannten NaCl/ PEG- Präzipitation bedient, wobei nachfolgend die Isolierung der Nukleinsäuren in an sich bekannter Weise über die Bindung an eine silikatische feste Phase realisiert wird. Inwieweit sich dieses Verfahren von der hinlänglich bekannten Methode der NaCl/ PEG- Präzipitation mit den bekannten Problemen abhebt, wird nicht ersichtlich. Darüber hinaus bedarf auch dieses Verfahren einer Kälteinkubation und einer zwanzigminütigen Zentrifugation. Das Verfahren soll es gestatten, virale Nukleinsäuren aus einer Probe von bis zu 10 ml zu isolieren. Eine weitere kommerziell verfügbare Variante, welche die Bearbeitung von bis zu 1 ml Probe erlauben soll, basiert auf der Anreicherung der viralen Nukleinsäuren unter Verwendung eines speziellen Detergenzes. Eine initiale Inkubation der Probe mit einem Lysereagenz führt dabei zur Lyse der Viren. Nachfolgend kommt es zur Ausbildung eines "Detergenz-Nukleinsäurekomplexes". Der Ansatz wird zentrifugiert und das erhaltene Pellet nachfolgend proteolytisch behandelt und die Nukleinsäure wiederum in an sich bekannter Art über die Anbindung an eine silikatische feste Phase isoliert (QIAamp UltraSens Virus Handbook). Auch hierbei wird auf Probleme mit der Resuspendierung des Pellets hingewiesen. Darüber hinaus erlaubt das Verfahren auch nur die Bearbeitung von Proben mit einem Volumen von maximal 1 ml.

Für die Isolierung zirkulierender zellfreier DNA aus großvolumigen Proben wird so verfahren, dass man alle Pufferkomponenten, welche für die Extraktion benötigt werden, aufskaliert. Dem Fachmann ist bekannt, dass damit ein enormer Aufwand an Reagenzien sowie an Zeit einhergeht.

Dem Fachmann wird auch ersichtlich, dass man für unterschiedliche Biomoleküle (z.B. Viren, oder DNA oder subzelluläre Partikel) immer auch unterschiedliche Verfahren benötigt, will man die Biomoleküle aus einer Probe aufkonzentrieren bzw. aufreinigen.

Ein völlig neuartiger Ansatz zur Aufkonzentrierung von Biomolekülen aus einer biologischen Probe und zur nachfolgenden Extraktion von Nukleinsäuren wird in der Patentschrift DE 10 2008 023 297 B4 offenbart.

Das Verfahren basiert auf der Verwendung von Polysaccharid-Derivaten für eine Komplexierung der in einer Probe enthaltenen Biomoleküle. Bei den Polysaccharid-Derivaten handelt es sich um die Salze von Polyuronsäuren. Besonders geeignet sind dabei die sog. Alginate der Alginsäuren. Bei Alginaten handelt es sich um strukturgebende Elemente bei Braunalgen. Alginat ist ein Polysaccharid, welches aus 1,4-verknüpfter-α-L-Guluronsäure (G) und β-D-Mannuronsäure (M) besteht.

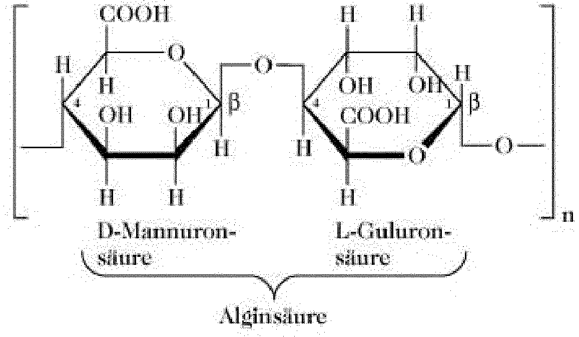

Es bildet homopolymere Bereiche, in denen Mannuronsäure oder Guluronsäure als Block vorliegt.

Alginate werden in der Lebensmittel- sowie der Pharma- und Kosmetikindustrie eingesetzt, z.B. als Geliermittel in der Lebensmittelindustrie, als Apreturmittel für Textilien, zur Herstellung von photographischen Papieren oder in der zahnmedizinischen Praxis zur Herstellung von Zahn- und Kieferabformungen.

Die Patentschrift beschreibt die Nutzung der Fähigkeit von Alginaten, in Lösungen mit niedrigem Kalziumgehalt zu gelieren und sog. Hydrogele zu bilden. Die Ursache der Gelierung begründet sich in der Einlagerung von Kalziumionen in die Zickzackstruktur der GG- Blöcke. Auf diese Zone lagert sich dann die Zickzackstruktur eines anderen Alginatmoleküls. Es kommt hierdurch zur Ausbildung dreidimensionaler Strukturen. Die Ausbildung von Gelen erfolgt auch in Kombination mit starken Säuren. Die entstandenen Gelstrukturen können darüber hinaus auch wieder spezifisch zerstört werden.

Unter Ausnutzung der Ausbildung von Alginatgelen kann die Anreicherung von Biomolekülen einfach und schnell erfolgen und nachfolgend die Isolierung von Nukleinsäuren durchgeführt werden.

Der Ablauf des Verfahrens ist wie folgt beschrieben:
1. Mischen einer wässrigen Alginatlösung mit einer flüssigen biologischen Probe
2. Zugabe einer wässrigen Lösung, welche die Gelausbildung/ Pelletbildung induziert (z.B. Verwendung einer 1 M Kalziumchloridlösung oder einer 1%igen Salzsäurelösung)
3. Mischen der Probe und kurze Inkubation bei Raumtemperatur
4. Zentrifugation der Probe und Entfernung des Überstandes
5. Auflösen des Pellets oder Gelstückchens und damit Freisetzung der Biomoleküle und ggf. nachfolgende direkte Isolierung von DNA oder RNA in an sich bekannter Art und Weise

Das in der Patentschrift offenbarte Verfahren ist extrem effizient und gestattet es, auch großvolumige Proben schnell und einfach für die Anreicherung von Biomolekülen einzusetzen.

Allerdings zeigt das beschriebene Verfahren einen großen Nachteil. Die Automatisierung des Prozesses ist schwierig durchzuführen. Dies begründet sich damit, dass der Anreicherungsschritt immer mittels Zentrifugation erfolgen muss. Bekannter Weise ist die Integration eines Zentrifugationsschrittes in einen automatisierten Prozess nicht einfach umzusetzen und erhöht darüber hinaus den Kostenaufwand für einen Automaten signifikant.

### Aufgabe der Erfindung

Der Erfindung lag die Aufgabe zugrunde, die Nachteile der bekannten technischen Lösungen zu beseitigen. Die vorliegende Erfindung wird durch die Ansprüche definiert.

### Lösung der Aufgabe

Die Aufgabe wurde gemäß den Merkmalen der Patentansprüche gelöst. Das erfindungsgemäße Verfahren erlaubt es, Biomoleküle (auch aus großvolumigen Proben) anzureichern und die Biomoleküle nachfolgend freizusetzen oder aus diesen ggf. Nukleinsäuren (DNA und RNA) zu isolieren bzw. die Nukleinsäuren freizusetzen.

Das Verfahren ermöglicht es, alle notwendigen Prozessschritte einfach und schnell in einen automatisierten Prozess zu implementieren. Überraschenderweise kann dies auf einem ganz einfachen Weg - ohne einen Zentrifugationsschritt - durchgeführt werden.

Das Verfahren basiert auf dem bekannten Verfahren, eine biologische flüssige Probe mit einer Alginatlösung und mit Salzen von di- bzw. polyvalenten Kationen (z.B. Kalzium-, Zink,- oder Aluminiumsalze) oder mit einer Säure zu versetzen. Die Anreicherung der Biomoleküle erfolgt nachfolgend aber nicht durch einen Zentrifugationsschritt. Der Probe werden magnetische oder paramagnetische Partikel zugegeben. Dabei spielt es keine Rolle, um welche Art von Partikeln es sich handelt. Sowohl die Größe der Partikel, als auch ihre Funktionalisierung ist von untergeordneter Bedeutung. Es können also Nanopartikel, Mikropartikel oder größere Partikel verwendet werden. Für eine einfache Abtrennung der Partikel sind magnetische Partikel am besten geeignet. In diesem Fall werden die Partikel mittels eines Magneten separiert und der Probenüberstand wird entfernt. Nach Entfernung des Probenüberstandes befinden sich die anzureichernden Biomoleküle (subzelluläre Partikel, Viren oder Nukleinsäuren) vollständig in einem Komplex aus Alginatgel und magnetischen bzw. paramagnetischen Partikeln. Diese Anreicherung erfolgte damit überraschenderweise ohne die in der Patentschrift DE 10 2008 023 297 B4 aufgeführte Zentrifugation. Die Freisetzung der komplexierten Biomoleküle kann jetzt dadurch erfolgen, dass Reagenzien zugesetzt werden, welche die Alginat-Gelstruktur wieder zerstören und damit die komplexierten Biomoleküle freisetzen. Dies kann z.B. mittels einer Pufferlösung erfolgen, welche einen Chelatbildner (EDTA) enthält oder aber auch mittels der Zugabe einer Lösung aus Tri-Natriumcitrat-Dihydrat. Nach Zugabe dieser Reagenzien erfolgt eine kurze Resuspendierung der Partikel und eine Inkubation. Die Inkubation dient der Auflösung der Alginat-Gelstruktur und der Freisetzung der Biomoleküle. Nachfolgend werden die magnetischen bzw. paramagnetischen Partikel durch Anlegen eines Magnetfeldes abgetrennt. Der resultierende Überstand enthält die Biomoleküle, welche nachfolgende dann aufgearbeitet oder analysiert werden können.

Damit benötigt das Verfahren keinen Zentrifugationsschritt und kann extrem einfach komplett automatisiert durchgeführt werden. Dies ermöglicht es erstmals, auch große Probenumfänge effizient und schnell zu bearbeiten.

Das erfindungsgemäße Verfahren zeigt noch einen weiteren Vorteil. In einer speziellen Ausführungsform kann man die für die Separation eingesetzten Partikel der Art auswählen, dass diese in der Lage sind, Nukleinsäuren zu binden. Das bedeutet, die Partikel dienen zum einen der Separation des Biomolekül-Alginat-Komplexes und nachfolgend auch der Extraktion hochreiner Nukleinsäuren. Dieses Verfahren ist insbesondere bedeutsam, wenn sogenannte zirkulierende zellfreie Nukleinsäuren aus einer zellfreien Probe angereichert und nachfolgend isoliert werden sollen. Dazu wird der Probe (Serum, Plasma, Urin etc.) eine Alginatlösung und eine wässrige Lösung, enthaltend Salze von di- bzw. polyvalenten Kationen (z.B. Kalziumchlorid oder Aluminiumchlorid) oder einer schwachen Säure zugegeben sowie paramagnetische oder magnetische Partikel. Diese Partikel werden so ausgewählt, dass sie unter spezifischen Bedingungen in der Lage sind, Nukleinsäuren zu binden. Solche Partikel sind dem Fachmann bekannt und werden in der Routine für die Isolierung von Nukleinsäuren eingesetzt. Nach Zugabe dieser Komponenten wird der Ansatz kurz gemischt und für einige Minuten bei Raumtemperatur inkubiert. Nachfolgend werden die Partikel mittels eines Magneten separiert und der Probenüberstand wird entfernt. Nach Entfernung des Überstandes werden die Partikel (der Alginat-Gel-Partikel-Komplex) mit einem Puffer versetzt, der eine Zerstörung des Alginat-Gel-Komplexes ermöglicht. Dazu werden die Partikel resuspendiert und der Ansatz für einige Minuten inkubiert. Dabei können dem Puffer auch weitere Zusätze wie proteolytische Enzyme etc. zugegeben werden. Die Inkubation kann bei Raumtemperatur erfolgen oder auch bei höheren Temperaturen, vorzugsweise 50°C - 70°C. Nach Freisetzung der komplexierten zellfreien Nukleinsäure bindet diese an die magnetischen oder paramagnetischen Partikel. Dies kann durch den eingesetzten Puffer erfolgen. Vorzugsweise effektiviert man die Anbindung der DNA durch die Zugabe eines Bindungspuffers. Als Puffer können Reagenzienkombinationen eingesetzt werden, die dem Fachmann aus der Patentschrift DE 10 2008 023 297 B4 bekannt sind. Wichtig ist, dass der erste Puffer eine Zerstörung der Alginat-Gelstruktur und eine nachfolgende Anbindung der freigesetzten DNA ermöglicht, allein oder in Kombination mit einem zusätzlichen Bindungspuffer.

Es ist ebenso möglich, neben dem Lysepuffer einen Bindungspuffer einzusetzen, der es erlaubt, eine Selektivität in Bezug auf die aufzureinigenden Nukleinsäuren zu erreichen (Größenfraktionierung oder Trennung von DNA und RNA). Der weitere Extraktionsprozess ist dem Fachmann bekannt. Die an den Partikeln gebundenen Nukleinsäuren werden gewaschen und die Nukleinsäuren werden final von den Partikeln durch Zugabe von Wasser oder durch Zugabe eines Niedrigsalzpuffers abgelöst.

Mittels des erfindungsgemäßen Verfahrens kann damit auch der gesamte Prozess, beginnend mit der Anreicherung von Biomolekülen bis hin zur finalen Extraktion hochreiner Nukleinsäuren, automatisiert durchgeführt werden. Es ist kein Zentrifugationsschritt mehr notwendig. Dies bedeutet einen entscheidenden Vorteil gegenüber dem in der Patentschrift DE 10 2008 023 297 B4 offenbarten Verfahren. Darüber hinaus kann mittels des erfindungsgemäßen Verfahrens de facto jedes Probenvolumen bearbeitet werden und ist damit nicht auf kleine Volumina limitiert und es ist darüber hinaus einfach zu automatisieren. Die Volumen der flüssigen Ausgangsproben können beliebig gewählt werden, was ein sehr breites Anwendungsspektrum erlaubt. Es kann z.B. mit Proben von 500 µl oder aber auch 10 ml gearbeitet werden. Damit ist eine enorm große Applikationsbreite gegeben.

Das erfindungsgemäße Verfahren ist auch geeignet, generell Nukleinsäuren aus einer Probe zu isolieren. Liegen die zu isolierenden Nukleinsäuren nicht als freie Nukleinsäuren vor, so kann die Probe in an sich bekannter Form lysiert werden. Die dadurch aus der Probe freigesetzten Nukleinsäuren werden danach wiederum entsprechend dem erfindungsgemäßen Verfahren isoliert. Auch hierbei kann man nach dem Auflösen der Alginat-Gelstruktur (welche die angereicherten Nukleinsäure enthält) eine "klassische" Aufreinigung der Nukleinsäuren durchführen oder die nach Auflösung der Alginat-Gelstruktur mittels eines basischen Niedrigsalzpuffers die erhaltene Lösung direkt für eine PCR- Reaktionen einsetzen.

Die Erfindung wird nachfolgend anhand von Ausführungsbeispielen näher erläutert.

### Ausführungsbeispiel 1: Anreicherung zellfreier DNA aus einer Plasmaprobe von 1 ml. Nachweis der notwendigen Kombination von Alginatlösung, Reagenz zur Ausbildung eines Alginatgels sowie mittels eines Magnetfeldes separierbarer Partikel

Ausgangsprobe für die Anreicherung war Humanplasma. Die Plasmaprobe wurde vor der Anwendung nochmals für 10 min zentrifugiert, um ggf. noch vorhandene Zellen zu entfernen. Weitergearbeitet wurde mit dem Überstand.
Es wurden drei verschiedene Verfahren getestet.

Probe 1: Der Probe wurden 30 µl einer 0.5%igen Alginatlösung zugegeben und der Ansatz kurz gemischt. Danach wurden 150 µl einer 1 molaren Kalziumchloridlösung dazugeben sowie 50 µl einer Magnetpartikelsuspension (MAG Suspension; Analytik Jena AG).

Probe 2: Der Probe wurden 30 µl einer 0.5%igen Alginatlösung zugegeben und der Ansatz kurz gemischt. Danach wurden 50 µl einer Magnetpartikelsuspension (MAG Suspension; Analytik Jena AG) zugebenen.

Probe 3: Der Probe wurden 150 einer 1 molaren Kalziumchloridlösung zugegeben und der Ansatz kurz gemischt. Danach wurden 50 µl einer Magnetpartikelsuspension (MAG Suspension; Analytik Jena AG) zugebenen.

Die Proben wurden dann wie folgt weiterbearbeitet.

Der Ansatz wurde kurz gemischt und für 10 Minuten inkubiert. Nachfolgend erfolgte die Separation der Magnetpartikel mittels eines Magneten. Der Überstand wurde entfernt und die Magnetpartikel wurden mit 1 ml Wasser gewaschen. Nach erneuter Separation der Magnetpartikel wurde der Überstand komplett entfernt.

Zum Komplex Magnetpartikel-Alginatgel-DNA wurde 400 µl Puffer (4 M Guanidinthiocyanat, EDTA) sowie 20 µl Proteinase K (20mg/ ml) zugegeben und der Ansatz mit einer Pipette resuspendiert. Nachfolgend erfolgte eine Inkubation für 15 min bei 70°C.

Dieser Schritt diente der Zerstörung des Partikel-Alginatgel-DNA-Komplexes und damit der Freisetzung der komplexierten DNA. Anschließend erfolgte die Zugabe von 400 µl eines Bindungspuffers (Isopropanol/ Triton X-100) und ein erneutes Mischen der Partikel sowie eine Inkubation für 2 min. Dieser Schritt diente der Bindung der DNA an die Partikel. Danach wurden die Magnetpartikel separiert und der Überstand wurde verworfen. Die Partikel wurden nachfolgend mit dem Fachmann bekannten alkoholischen Waschpuffern gewaschen und final getrocknet. Im letzten Schritt wurde die gebundene DNA durch Zugabe von 50 µl H₂O von den Partikeln abgelöst und in ein neues Reaktionsgefäß überführt.

Der Nachweis der Anreicherung und nachfolgenden Extraktion von zellfreier DNA erfolgte mittels real Time PCR. Amplifiziert wurde dazu eine humanspezifische Targetsequenz Östrogenrezeptor 1.

### Protokoll real Time PCR zur Amplifikation der humanspezifischen Targetsequenz

### (Östrogenrezeptor 1).

sense Primer (5'- CGC CGC CAA CGC GCA GGT CTA-3')
antisense Primer (5'- AGC CGA ACG CCG CAG CCT CA-3')
1 Sonde (5'-FAM CCT CCC CTA CGG CCC CGG G-BHQ1-3')

### Reaktionsansatz

### Pro Probe:

| | |
|---|---|
| sense Primer (50 pmol/ µl) | 0,1 µl |
| antisense Primer (50 pmol/ µl) | 0,1 µl |
| Sonde (25 pmol/ µl) | 0,1 µl |
| dNTP- Mix (12,5 mM) | 0,3 µl |
| 10X PCR- Buffer (MgCl₂ included) | 1,5 µl |
| Taq- DNA- Polymerase | 0,75 U |
| PCR- Grade H₂O | add 15 µl |

### Amplifikations-/ Hybridisierungskonditionen

| Schritt 1: | | | | |
|---|---|---|---|---|
| | Denaturierung | | 95°C | 120" |

| Schritt 2 | | | | |
|---|---|---|---|---|
| | Amplifizierung | 45 Zyklen | | |
| | | | 95°C | 4" |
| | (Messung) | | 65°C | 45" |

### Ergebnis PCR

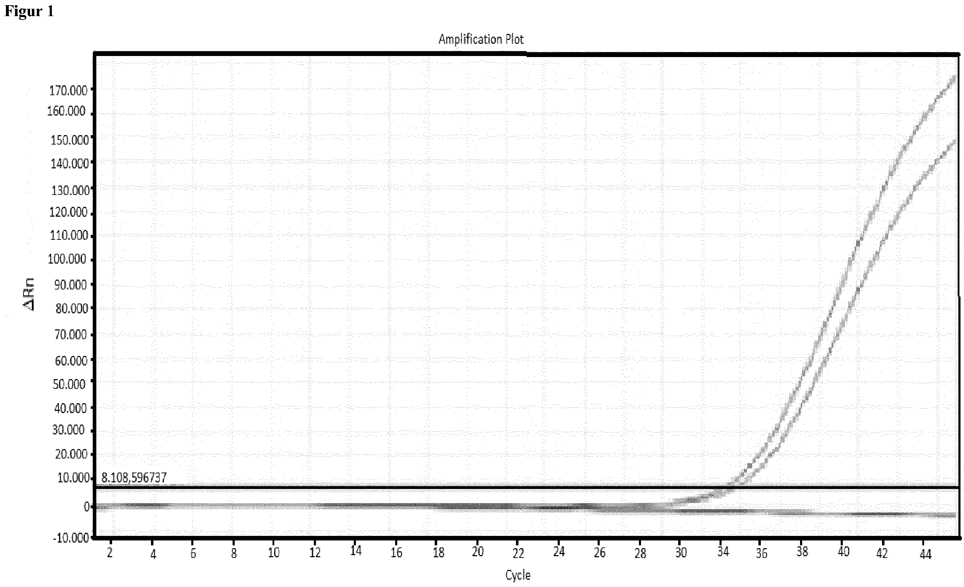

Die Ergebnisse in Figur 1 zeigen, dass nur die Kombination von Alginatlösung, Reagenz zur Ausbildung eines Alginatgels sowie mittels eines Magnetfeldes separierbarer Partikel eine Anreicherung und nachfolgende Extraktion von DNA ermöglicht (schwarze Kurven). Damit ist bewiesen, dass der erste Schritt die Komplexierung der freien DNA mit dem sich ausbildendenden Alginatgel ist und die zugegebenen Partikel sich mit diesem Komplex verbinden. Damit kann dann durch das Anlegen eines Magnetfeldes der Partikel-Alginatgel- DNA- Komplex separiert werden. Eine Zentrifugation, wie dies in der Patentschrift DE 102008023297B4 beschrieben wird, ist somit nicht erforderlich.

### Ausführungsbeispiel 2: Anreicherung zellfreier DNA aus einer Plasmaprobe von 1 ml sowie von 5 ml und nachfolgende Extraktion der DNA

Ausgangsprobe für die Anreicherung war Humanplasma. Die Plasmaprobe wurde vor der Anwendung nochmals für 10 min zentrifugiert, um ggf. noch vorhandene Zellen zu entfernen. Weitergearbeitet wurde mit dem Überstand.

Die 1 ml Probe wurde wie folgt behandelt. Der Probe wurden 30 µl einer 0.5%igen Alginatlösung zugegeben und der Ansatz kurz gemischt. Danach wurden 150 µl einer 1 molaren Kalziumchloridlösung dazugeben sowie 50 µl einer Magnetpartikelsuspension (MAG Suspension; Analytik Jena AG). Der Ansatz wurde kurz gemischt und für 10 Minuten inkubiert. Nachfolgend erfolgte die Separation der Magnetpartikel mittels eines Magneten. Der Überstand wurde entfernt und die Magnetpartikel wurden mit 1 ml Wasser gewaschen. Nach erneuter Separation der Magnetpartikel wurde der Überstand komplett entfernt.

Zum Komplex Magnetpartikel-Alginatgel-DNA wurde 400 µl Puffer (4 M Guanidinthiocyanat, EDTA) sowie 20 µl Proteinase K (20mg/ ml) zugegeben und der Ansatz mit einer Pipette resuspendiert. Nachfolgend erfolgte eine Inkubation für 15 min bei 70°C.

Dieser Schritt diente der Zerstörung des Partikel-Alginatgel-DNA-Komplexes und damit der Freisetzung der komplexierten DNA. Anschließend erfolgte die Zugabe von 400 µl eines Bindungspuffers (Isopropanol/ Triton X- 100) und ein erneutes Mischen der Partikel sowie eine Inkubation für 2 min. Dieser Schritt diente der Bindung der DNA an die Partikel. Danach wurden die Magnetpartikel separiert und der Überstand wurde verworfen. Die Partikel wurden nachfolgend mit dem Fachmann bekannten alkoholischen Waschpuffern gewaschen und final getrocknet. Im letzten Schritt wurde die gebundene DNA durch Zugabe von 50 µl H₂O von den Partikeln abgelöst und in ein neues Reaktionsgefäß überführt.

Die 5 ml Probe wurde wie folgt behandelt. Die Probe wurde in ein 15 ml Reaktionsgefäß überführt. Der Probe wurden 150 µl einer 0.5%igen Alginatlösung zugegeben und der Ansatz kurz gemischt. Danach wurden 600 µl einer 1 molaren Kalziumchloridlösung dazugeben sowie 100 µl einer Magnetpartikelsuspension (MAG Suspension; Analytik Jena AG). Der Ansatz wurde kurz gemischt und für 10 Minuten inkubiert. Nachfolgend erfolgte die Separation der Magnetpartikel mittels eines Magneten. Der Überstand wurde entfernt und die Magnetpartikel wurden mit 5 ml Wasser gewaschen. Nach erneuter Separation der Magnetpartikel wurde der Überstand komplett entfernt.

Zum Komplex Magnetpartikel-Alginatgel-DNA wurde 400 µl Puffer (4 M Guanidinthiocyanat, EDTA) sowie 20 µl Proteinase K (20mg/ ml) zugegeben, resuspendiert und der Ansatz mit einer Pipette in ein 1,5 ml Reaktionsgefäß überführt. Nachfolgend erfolgte eine Inkubation für 15 min bei 70°C.

Dieser Schritt diente der Zerstörung des Partikel-Alginatgel-DNA-Komplexes und damit der Freisetzung der komplexierten DNA. Anschließend erfolgte die Zugabe von 400 µl eines Bindungspuffers (Isopropanol/ Triton X- 100) und ein erneutes Mischen der Partikel sowie eine Inkubation für 2 min. Dieser Schritt diente der Bindung der DNA an die Partikel. Danach wurden die Magnetpartikel separiert und der Überstand wurde verworfen. Die Partikel wurden nachfolgend mit dem Fachmann bekannten alkoholischen Waschpuffern gewaschen und final getrocknet. Im letzten Schritt wurde die gebundene DNA durch Zugabe von 50 µl H₂O von den Partikeln abgelöst und in ein neues Reaktionsgefäß überführt.

Der Nachweis der Anreicherung und nachfolgenden Extraktion von zellfreier DNA erfolgte mittels real Time PCR. Amplifiziert wurde dazu eine humanspezifische Targetsequenz (Östrogenrezeptor 1).

### Ergebnisse real Time PCR

Protokoll real Time PCR zur Amplifikation der humanspezifischen Targetsequenz (Östrogenrezeptor 1). Protokoll siehe Beispiel 1.

| **Probe** | **Ct- Werte** |
|---|---|
| 1 ml Probe (rote Kurve) | 35/ 34,95 |
| 5 ml Probe (schwarze Kurve ) | 32,25/ 32,30 |

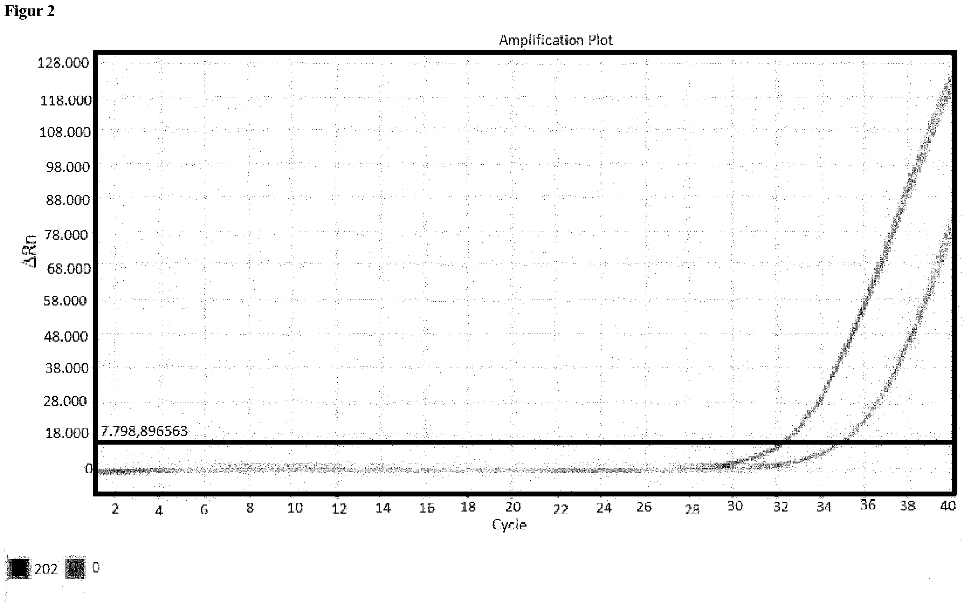

Wie die Ergebnisse in Figur 2 zeigen, wird aus der 5 ml Probe mehr DNA angereichert und nachfolgend isoliert, als aus der 1 ml Probe. Damit eignet sich das erfindungsgemäße Verfahren auch für die Anreicherung und Extraktion von zellfreier DNA aus großvolumigen Proben.

### Ausführungsbeispiel 3: Anreicherung von genomischer DNA aus einer wässrigen Lösung (1 ml) und direkte Freisetzung der genomischen DNA ohne eine weitere DNA-Extraktion. Vergleich des erfindungsgemäßen Verfahrens mit dem Verfahren aus der Patentschrift DE 102008023297 B4

Ausgangsprobe für die Anreicherung war eine wässrige Lösung, welche genomische DNA enthielt. Die Anreicherung erfolgte mittels des Verfahrens aus der Patentschrift DE 102008023297 B4 sowie mittels des erfindungsgemäßen Verfahrens ohne einen Zentrifugationsschritt. Für das Verfahren aus der Patentschrift wurde das kommerzielle Produkt PME free-circulating DNA Extraction Kit (Analytik Jena AG) eingesetzt). Das erfindungsgemäße Verfahren wurde wie folgt durchgeführt.

Der Probe wurden 30 µl einer 0.5%igen Alginatlösung zugegeben und der Ansatz kurz gemischt. Danach wurden 150 µl einer 1 molaren Kalziumchloridlösung sowie 50 µl einer Magnetpartikelsuspension (MAG Suspension; Analytik Jena AG) dazugegeben. Der Ansatz wurde kurz gemischt und für 5 Minuten inkubiert. Nachfolgend erfolgte die Separation der Magnetpartikel mittels eines Magneten. Der Überstand wurde entfernt und die Magnetpartikel wurden mit 1 ml Wasser gewaschen. Nach erneuter Separation der Magnetpartikel wurde der Überstand komplett entfernt. Der Komplex Magnetpartikel-Alginatgel-DNA wurde nachfolgend zerstört, um die DNA freizusetzen. Dazu wurden 50 µl einer 50 mM Natriumcitratlösung zu den Partikeln gegeben und der Ansatz mit einer Pipette resuspendiert. Nach kurzer Inkubation wurden die Magnetpartikel separiert und der Überstand wurde in ein neues Gefäß überführt und nachfolgend noch mit 50 µl Wasser versetzt. Die Überprüfung, ob die genomische DNA der Probe auch ohne Zentrifugation angereichert wurde, erfolgte auf einem Agarosegel. Wie die gelelektrophoretische Darstellung zeigt, konnten mit beiden Verfahren die genomische DNA aus der 1 ml Probe aufkonzentriert werden. Dabei besticht das erfindunsgemäße Verfahren durch seine Einfachheit, da keine Zentrifugationschritte mehr benötigt wurden.

### Ausführungsbeispiel 4: Anreicherung von Viren und nachfolgende Extraktion der viralen Nukleinsäure

Ausgangsprobe für die Anreicherung war Humanplasma. Der Plasmaprobe wurde inaktiviertes Gelbfiebervirus zugegeben. Für die Anreicherung der Viren wurde 1 ml Probe eingesetzt. Die 1 ml Probe wurde wie folgt behandelt. Der Probe wurden 30 µl einer 0.5%igen Alginatlösung zugegeben und der Ansatz kurz gemischt. Danach wurden 150 µl einer 1 molaren Kalziumchloridlösung dazugeben sowie 50 µl einer Magnetpartikelsuspension (MAG Suspension; Analytik Jena AG). Der Ansatz wurde kurz gemischt und für 10 Minuten inkubiert. Nachfolgend erfolgte die Separation der Magnetpartikel mittels eines Magneten. Der Überstand wurde entfernt und die Magnetpartikel wurden mit 1 ml Wasser gewaschen. Nach erneuter Separation der Magnetpartikel wurde der Überstand komplett entfernt.

Zum Komplex Magnetpartikel-Alginatgel-DNA wurde 400 µl Puffer (4 M Guanidinthiocyanat, EDTA) sowie 20 µl Proteinase K (20mg/ ml) zugegeben und der Ansatz mit einer Pipette resuspendiert. Nachfolgend erfolgte eine Inkubation für 15 min bei 60°C.

Dieser Schritt diente der Zerstörung des Partikel-Alginatgel-Virus-Komplexes und damit der Freisetzung der komplexierten Viren und der Zerstörung zur Freisetzung der viralen Nukleinsäure (virale RNA). Anschließend erfolgte die Zugabe von 400 µl eines Bindungspuffers (Guanidinthiocyanat/ Isopropanol/ Triton X- 100) und ein erneutes Mischen der Partikel sowie eine Inkubation für 2 min. Dieser Schritt diente der Bindung der viralen RNA an die Partikel. Danach wurden die Magnetpartikel separiert und der Überstand wurde verworfen. Die Partikel wurden nachfolgend mit dem Fachmann bekannten alkoholischen Waschpuffern gewaschen und final getrocknet. Im letzten Schritt wurde die gebundene DNA durch Zugabe von 50 µl H₂O von den Partikeln abgelöst und in ein neues Reaktionsgefäß überführt.

Der Nachweis der Anreicherung der Viruspartikel und der nachfolgenden Extraktion der viralen RNA erfolgte mittels einer Gelbfiebervirus- spezifischen real Time PCR.

### Ergebnisse real Time PCR

Protokoll real Time PCR zur Amplifikation der 5' Noncoding Region von Yellow Fever Virus
YFallF: (5'-GCT AAT TGA GGT GYA TTG GTC TGC-3')
YFallR (5'-CTG CTA ATC GCT CAA MGA ACG-3')
YFallP (5'-FAM-ATC GAG TTG CTA GGC AAT AAA CAC-BHQ1-3')

### Reaktionsansatz

### Pro Probe:

| | |
|---|---|
| YFallF (50 pmol/ µl) | 0,1 µl |
| YFallR (50 pmol/ µl) | 0,1 µl |
| YFallP (25 pmol/ µl) | 0,1 µl |
| dNTP- Mix (12,5 mM) | 0,3 µl |
| 10X PCR- Buffer (MgCl₂ included) | 1,5 µl |
| Taq- DNA- Polymerase | 0,75 U |
| PCR- Grade H₂O | add up to 15 µl |

### Amplifikations-/ Hybridisierungskonditionen

| Schritt 1: | | | | |
|---|---|---|---|---|
| | Denaturierung | | 95°C | 120 sec |

| Schritt 2 | | | | |
|---|---|---|---|---|
| | Amplifizierung | 45 Zyklen | | |
| | | | 95°C | 4 sec |
| | (Messung) | 65°C | | 45 sec |

### Ergebnisse PCR

| **Probe** | **Ct-Werte** |
|---|---|
| 1 ml Probe (blaue) | 28,8/ 28,9 |

## Patentansprüche

1. Verfahren zur Anreicherung von Biomolekülen und zur Entfernung der Biomoleküle aus einer biologischen Probe, **dadurch gekennzeichnet, dass** - in Anwesenheit von magnetischen oder paramagnetischen Partikeln - eine biologische Probe mit einer Alginatlösung und mit Salzen von di- bzw. polyvalenten Kationen oder mit einer Säure versetzt wird, wobei sich an den Partikeln ein Alginatgel-Biomolekül-Komplex bildet, dieser durch Separation der Partikel von der Probe entfernt wird und aus dem anschließend die Biomoleküle oder Inhaltstoffe der Biomoleküle freigesetzt werden.

2. Verfahren nach Anspruch 1, **dadurch gekennzeichnet, dass** als Salze von di- bzw. polyvalenten Kationen Kalzium-, Zink- oder Aluminiumsalze verwendet werden.

3. Verfahren nach einem der Ansprüche 1 oder 2, **dadurch gekennzeichnet, dass** es sich bei den Biomolekülen um zellfreie Nukleinsäuren, Viren oder subzelluläre Mikropartikel handelt.

4. Verfahren nach einem der Ansprüche 1 bis 3, **dadurch gekennzeichnet, dass** im Fall von magnetischen Partikeln die Separation der Partikel mit dem Alginatgel-Biomolekül-Komplex über einen Magneten erfolgt.

5. Verfahren nach einem der Ansprüche 1 bis 4, **dadurch gekennzeichnet, dass** die Freisetzung der Biomoleküle durch Auflösung des sich an den Partikeln befindenden Alginatgel-Biomolekül-Komplex erfolgt, wobei die Partikel und die Biomoleküle frei werden.

6. Verfahren nach Anspruch 5, **dadurch gekennzeichnet, dass** die Auflösung des Alginatgel-Biomolekül-Komplexes durch Tri-Natriumcitrat-Dihydrat oder durch Chelatbildner, vorzugsweise EDTA, erfolgt.

7. Verfahren nach Anspruch 5 oder 6, **dadurch gekennzeichnet, dass** nach der Auflösung des sich an den Partikeln befindenden Alginatgel-Biomolekül-Komplexes eine Resuspendierung der Partikel und eine Inkubation erfolgt und die Partikel abgetrennt werden.

8. Verfahren nach einem der Ansprüche 1 bis 7 zur Anreicherung von Nukleinsäuren und zur Entfernung der Nukleinsäuren aus einer biologischen Probe, **dadurch gekennzeichnet, dass** die Partikel, an denen sich der Alginatgel-Nukleinsäure-Komplex gebildet hat, nach dessen Auflösung gleichzeitig zur Anbindung der aus dem Komplex freigesetzten Nukleinsäuren dienen.

9. Verfahren nach Anspruch 8, **dadurch gekennzeichnet, dass** nach der Auflösung des Alginatgel-Nukleinsäure-Komplexes und der Resuspendierung der Partikel ein Bindungspuffer zur Anbindung der Nukleinsäuren an die Partikel zugegeben wird und die nachfolgende Isolierung der Nukleinsäuren in bekannte Weise erfolgt.

10. Kit zur Durchführung des Verfahrens gemäß einem der Ansprüche 1 bis 9, umfassend
a) eine Alginatlösung
b) mindestens ein Salz von di- bzw. polyvalenten Kationen oder eine Säure
c) Partikel
d) Mittel zur Abtrennung der Partikel, die mit Biomolekülen einen Komplex aus a) und b) gebildet haben
e) Mittel zu Freisetzung von Biomolekülen, die in einem Komplex aus a), b) und c) gebunden sind, vorzugsweise Chelatbildner

## Claims

1. A method for enriching biomolecules and for removal of the biomolecules from a biological sample, **characterized in that** - in the presence of magnetic or paramagnetic particles - an alginate solution and salts of divalent and/or polyvalent cations or an acid is added to a biological sample, wherein an alginate-gel-biomolecule-complex is formed on the particles, said complex is removed from the sample by separation of the particles, and from which subsequently the biomolecules or contents of the biomolecules are released.

2. The method according to claim 1, **characterized in that** calcium, zinc or aluminium salts are used as salts of divalent and/or polyvalent cations.

3. The method according to any one of claims 1 or 2, **characterized in that** the biomolecules are cell-free nucleic acids, viruses or subcellular microparticles.

4. The method according to any one of claims 1 to 3, **characterized in that** in the case of magnetic particles, separation of the particles with the alginate-gel-biomolecule-complex takes place via a magnet.

5. The method according to any one of claims 1 to 4, **characterized in that** release of the biomolecules takes place by dissolving of the alginate-gel-biomolecule-complex, which is located on the particles, wherein the particles and the biomolecules are released.

6. The method according to claim 5, **characterized in that** dissolving of the alginate-gel-biomolecule-complex takes place by trisodium citrate dihydrate or by chelating agents, preferably EDTA.

7. The method according to claim 5 or 6, **characterized in that** after dissolving of the alginate gel-biomolecule-complex located on the particles, resuspension of the particles and an incubation takes place, and the particles are separated.

8. The method according to any one of claims 1 to 7 for enriching nucleic acids and for removal of the nucleic acids from a biological sample, **characterized in that** the particles on which the alginate-gel-nucleic acid-complex has formed, after its dissolving serve at the same time for binding of the nucleic acids released from the complex.

9. The method according to claim 8, **characterized in that** after dissolving of the alginate-gel-nucleic acid-complex and resuspension of the particles, a binding buffer for binding the nucleic acids to the particles is added, and subsequent isolation of the nucleic acids takes place in a known manner.

10. Kit for carrying out the method according to any one of claims 1 to 9, comprising
a) an alginate solution
b) at least one salt of divalent and/or polyvalent cations or an acid
c) particles
d) means for separating the particles which have formed a complex from a) and b) with biomolecules
e) means for the release of biomolecules which are bound in a complex of a), b) and c), preferably chelating agents.

## Revendications

1. Procédé d'enrichissement des biomolécules et d'élimination des biomolécules à partir d'un échantillon biologique, **caractérisé en ce que** - en présence de particules magnétiques ou paramagnétiques - une solution d'alginate et des sels de cations divalents et/ou polyvalents ou un acide est ajouté à l'échantillon biologique, dans lequel un complexe gel d'alginate-biomolécules se forme sur les particules, ce complexe est éliminé de l'échantillon par séparation des particules et à partir duquel les biomolécules ou des substances contenues dans les biomolécules sont ensuite libérées.

2. Procédé selon la revendication 1, **caractérisé en ce que** l'on utilise comme sels de cations divalents et/ou polyvalents des sels de calcium, de zinc ou d'aluminium.

3. Procédé selon l'une des revendications 1 ou 2, **caractérisé en ce que** les biomolécules sont des acides nucléiques acellulaires, des virus ou des microparticules subcellulaires.

4. Procédé selon l'une des revendications 1 à 3, **caractérisé en ce que**, dans le cas de particules magnétiques, la séparation des particules avec le complexe gel d'alginate-biomolécule est effectuée par l'intermédiaire d'un aimant.

5. Procédé selon l'une des revendications 1 à 4, **caractérisé en ce que** la libération des biomolécules se fait par dissolution du complexe gel d'alginate-biomolécule présent sur les particules, libérant ainsi les particules et les biomolécules.

6. Procédé selon la revendication 5, **caractérisé en ce que** la dissolution du complexe alginate-gel-biomolécule est effectuée par du citrate trisodique dihydraté ou par des agents chélateurs, de préférence de l'EDTA.

7. Procédé selon la revendication 5 ou 6, **caractérisé en ce qu'**après la dissolution du complexe gel d'alginate-biomolécule se trouvant sur les particules, une remise en suspension des particules et une incubation ont lieu et les particules sont séparées.

8. Procédé selon l'une des revendications 1 à 7 pour l'enrichissement d'acides nucléiques et pour l'élimination des acides nucléiques à partir d'un échantillon biologique, **caractérisé en ce que** les particules, sur lesquelles le complexe gel d'alginate-acide nucléique s'est formé, servent simultanément, après sa dissolution, à la fixation des acides nucléiques libérés du complexe.

9. Procédé selon la revendication 8, **caractérisé en ce qu'**après la dissolution du complexe gel d'alginate-acide nucléique et la remise en suspension des particules, un tampon de liaison est ajouté pour lier les acides nucléiques aux particules et l'isolement ultérieur des acides nucléiques est effectué de manière connue.

10. Kit pour la mise en oeuvre du procédé selon l'une quelconque des revendications 1 à 9, comprenant
a) une solution d'alginate
b) au moins un sel de cations divalents et/ou polyvalents ou un acide
c) des particules
d) des moyens pour la séparation des particules ayant formé avec des biomolécules un complexe de a) et b)
e) des moyens pour libérer des biomolécules liées dans un complexe de a), b) et c), de préférence des agents chélateurs.
